# FASCICULE DE BREVET EUROPEEN

(11) **EP 0 425 391 B1**
(45) Date de publication et mention de la délivrance du brevet: **08.06.1994**
(21) Numéro de dépôt: 90403042.6
(22) Date de dépôt: 26.10.1990
(51) Int. Cl.: A61K 35/78, A23L 1/00, A61K 7/00

(54) **Compositions à base de suc et de protoplastes de végétaux, leur procédé d'obtention et leurs utilisations, notamment dans le domaine de la phytothérapie**
Pflanzlichen Saft und Protoplasten enthaltende Zusammensetzungen, Verfahren zu ihrer Herstellung und ihre Verwendungen, insbesondere auf dem Gebiet der Phytotherapie
Compositions comprising juice and plant protoplasts, process to prepare them and their application, especially in the field of phytotherapy

(30) Priorité: 27.10.1989 FR 8914186
(43) Date de publication de la demande: 02.05.1991
(73) Titulaire: INSTITUT DES SUBSTANCES VEGETALES, F-63960 Veyre-Monton (FR)
(72) Inventeur: Jean, Daniel, F-63270 Vic le Comte (FR)
(74) Mandataire: Gutmann, Ernest

(56) Documents cités:
- EP-A- 420 729
- FR-A- 977 029
- FR-A- 2 443 265

## Description

La présente invention concerne des compositions à base de protoplastes de végétaux, ainsi que leur procédé d'obtention et leurs utilisations, notamment dans le domaine de la phytothérapie tant par voie interne que par voie externe.

Deux types principaux de produits sont actuellement utilisés, en phytothérapie à savoir
- ceux qui font intervenir la plante entière sèche ou fraîche tels que les plantes séchées pour tisanes ou dispensées en gélules ou comprimés par exemple, et
- les extraits de plantes obtenus à l'état sec tels que les nébulisats, ou sous forme liquide tels que les teintures et les extraits fluides.

Les plantes entières présentent l'inconvénient de posséder une faible concentration en principes actifs mais l'avantage de proposer la totalité de ces derniers.

Les extraits présentent l'inconvénient de ne comporter qu'une partie des composants de la plante mais ont l'avantage de posséder une forte concentration en principes actifs quand ceux-ci sont présents et ce en particulier dans les formes sèches de ces extraits.

La présente invention a précisément pour but de proposer des compositions végétales présentant l'avantage non seulement de comporter l'ensemble des principes actifs de la plante mais également de posséder une très forte concentration en ces principes actifs.

L'invention propose également une méthode particulièrement efficace et simple à mettre en oeuvre pour l'obtention de telles compositions.

L'invention a plus particulièrement pour objet une composition végétale sèche issue d'une plante, cette composition étant exempte des divers constituants des membranes cellulosiques entourant les cellules végétales, en particulier de cellulose, et formée de protoplastes en mélange avec des constituants hydrosolubles appartenant au suc de cette plante, cette composition permettant de reconstituer l'essentiel du suc du végétal dont elle est issue lorsqu'elle est mise en suspension dans de l'eau.

En effet la cellule végétale a la particularité que ne possède pas la cellule animale d'être entourée d'une membrane cellulosique qui représente un poids important dans la plante. Cette membrane ne présente généralement pas d'intérêt thérapeutique à cause de son inertie chimique.

La composition végétale selon l'invention est plus particulièrement caractérisée:
- en ce qu'elle comprend des protoplastes, d'un diamètre compris entre 0,45»m et 100»m, insolubles dans l'eau, ces protoplastes étant eux-mêmes constitués du noyau de la cellule végétale et de cytoplasme cellulaire,
- en ce que la teneur en protoplastes est d'environ 10 à 20% en poids sec, les 90 à 80% restant étant représentés par des produits hydrosolubles habituellement contenus dans les sucs de plantes, notamment des sucres, des sels minéraux, des protéines etc...

L'invention a également pour objet un procédé d'obtention d'une composition selon l'invention caractérisé en ce qu'il comprend:
- un premier broyage de la plante entière, ou de parties de cette plante (tiges, racines, feuilles etc...) à une température comprise entre -10°C et -40°C, de préférence à environ -25°C,
- un second broyage des fractions de plantes obtenues lors de l'étape de broyage précédente, à une température comprise entre -40°C et -100°C, de préférence à environ -70°C,
- le tamisage des fractions obtenues lors de l'étape de broyage précédente, maintenues à une température comprise entre -40°C et -100°C, à l'aide de tamis de granulométrie variant d'environ 100»m à environ 500»m, suivi de la récupération des fractions passées à travers ce tamis,
- le pressage des fractions récupérées à l'étape précédente, et ramenées à une température de 0°C ± 5°C; les fractions pressées sont ensuite avantageusement soumises au cycle d'opérations suivantes:
   . congélation à une température comprise entre -10°C et -40°C, de préférence à environ -25°C,
   . mise en suspension de ces fractions congelées dans une quantité d'eau approximativement égale à la quantité de liquide obtenu lors de l'étape de pressage précédente,
   . pressage des fractions en suspension dans l'eau et ramenées à une température de 0°C ± 5°C,
   ce cycle étant répété entre 2 et 6 fois, de préférence 4 fois,
- la filtration des quantités de liquides obtenus lors de chacune des étapes de pressage précédentes, ces quantités étant, le cas échéant, réunies en une solution unique, sur un filtre ayant une porosité de 100»m, et récupération des filtrats (ou du filtrat)
- la concentration des filtrats (ou du filtrat) par une méthode d'élimination de l'eau à froid, notamment par osmose inverse, à la température de 0°C ± 5°C,
- avantageusement, la congélation des solutions (ou de la solution) concentrées obtenues à l'étape précédente, à une température de -10°C à -40°C, notamment de l'ordre de -25°C, suivie d'une étape de lyophilisation ce qui conduit à l'obtention d'une composition selon l'une des revendications 1 à 3 sous forme de poudre sèche.

Avantageusement la plante végétale mise en oeuvre dans ce procédé est fraîchement récoltée, c'est-à-dire depuis moins de 24 heures, et est congelée étalée en couches minces en chambre froide à -25°C environ.

Lors de la première étape de broyage du procédé décrit ci-dessus, la plante est coupée grossièrement, notamment à l'aide d'un broyeur à couteaux horizontaux. Cette opération est réalisée à -25°C environ dans l'enceinte de la chambre froide et en régulant la température dans le broyeur avec une addition d'azote liquide.

La seconde étape de broyage du procédé de l'invention, ou encore étape de cryobroyage proprement dit, est avantageusement réalisée en portant la plante, précédemment coupée, à une température de -70°C environ dans un tunnel où l'on introduit de l'azote liquide et dans lequel la plante est acheminée par une vis d'archimède. Les fractions de plante obtenues lors du premier broyage sont ainsi conduites jusqu'à un broyeur à marteaux lui-même refroidi à environ -70°C par de l'azote liquide. Ce second broyage est avantageusement effectué au-dessus d'un tamis de granulométrie variant d'environ 100»m à environ 500»m. Les fractions de plante obtenues lors du second broyage tombent directement dans ce tamis, et l'on récupère les fractions passées à travers le tamis.

Les fractions ainsi récupérées sont portées sans intervention extérieure à la température de 0°C ± 5°C puis l'étape de pressage du procédé de l'invention est avantageusement réalisée dans une presse à vis jusqu'à une pression ne permettant pas d'exclure de liquide de façon significative. Le liquide, ou la suspension, est recueillie et, après passage sur un tamis de porosité 100»m, est placée en congélation en couches de 2cm dans des plateaux. Conformément au cycle d'opérations décrit dans le procédé de l'invention, les fractions de plante pressées sont recongelées à -25°C par passage dans le même tunnel que celui décrit précédemment par aspersion d'azote liquide. On recommence alors la même opération de pressage que ci-dessus mais après avoir ajouté à la plante une quantité d'eau de préférence purifiée égale à la quantité de liquide exprimé lors du premier pressage.

Cette succession de congélation-décongélation-pressage est recommencée en tout quatre fois avec à chaque fois addition d'une quantité d'eau purifiée de préférence égale à la quantité de liquide obtenue lors du pressage précédent.

L'étape de concentration du procédé de l'invention est avantageusement réalisée par osmose inverse, de préférence à froid afin de ne pas dénaturer les composants végétaux contenus dans les liquides obtenus lors des étapes de pressage. L'opération est réalisée à 0°C ± 5°C par passage permanent des liquides sur un échangeur dans lequel circule du fluide réfrigérant. L'osmose est réalisée à environ 40 bars sur une membrane dont le seuil de rétention dans l'eau pure vis-à-vis du chlorure de sodium est de 99,9%.

Le volume final représente environ le dixième du volume initial.

Avantageusement, les suspensions sont toutes congelées en couches minces de 2cm et sont ensuite lyophilisées. La poudre sèche recueillie est ensuite broyée finement et tamisée selon l'usage qui doit en être fait.

Les compositions de l'invention se présentent donc sous forme de solutions, notamment aqueuses, plus ou moins concentrées en constituants végétaux, ou avantageusement sous forme d'un lyophilisat de poudre sèche.

L'étape de lyophilisation du procédé de l'invention est particulièrement avantageuse en ce qu'elle permet d'obtenir une poudre sèche (lyophilisat) dans laquelle l'intégrité des protoplastes est conservée.

A titre illustratif, la teneur en protoplastes d'une composition de l'invention peut être déterminée de la manière suivante:
- mise en suspension dans l'eau d'une quantité aliquote de ladite composition se présentant sous forme de poudre,
- filtration sur un filtre de porosité 0,45»m et récupération de la fraction insoluble dans l'eau qui reste sur le filtre,
- le cas échéant, étude au microscope de la fraction sus-mentionnée afin de vérifier qu'elle est bien essentiellement constituée de protoplastes,
- séchage de la fraction restée sur le filtre, et détermination du poids de cette fraction ainsi séchée par rapport au poids de la poudre de départ.

L'invention a également pour objet des compositions pharmaceutiques ou cosmétiques, comprenant une composition végétale selon l'invention, ou un mélange de plusieurs de ces compositions issues de végétaux différents, le cas échéant en association avec un véhicule physiologiquement acceptable.

A ce titre l'invention concerne plus particulièrement des compositions pharmaceutiques, ou cosmétiques, se présentant sous forme de gélules, ou de comprimés pour l'administration orale, ou encore sous forme de gels, d'émulsions ou de pommages pour un usage topique.

L'invention vise également une méthode de traitement esthétique comprenant l'administration, notamment par voie topique ou orale, d'une composition cosmétique de l'invention.

L'invention concerne également des compositions alimentaires ou diététiques comprenant une (ou plusieurs) composition de l'invention, le cas échéant en association avec un véhicule alimentaire.

Toute plante dont le suc est susceptible de contenir des constituants actifs dans le domaine de la phytothérapie, de la cosmétologie, ou encore de la diététique (notamment les plantes officinales ou médicinales) est utilisable dans le cadre de la présente invention.

L'invention sera plus particulièrement illustrée à l'aide des exemples de mise en oeuvre du procédé d'obtention de compositions de l'invention qui suivent, étant bien entendu que ces exemples ne sont nullement limitatifs.

### - Exemple 1

200kg de parties aériennes fraiches de Equisetum arvense sont récoltés et placés en couches de 5cm environ dans une chambre froide à -25°C. Après 24 heures, la plante (ou drogue) est broyée dans un broyeur à couteaux pour donner des morceaux de 1 à quelques cm. Puis la drogue coupée est admise dans le cryobroyeur et amenée à une température de -70°C. La température du broyeur est régulée à -85°C et le tamis possède une porosité de 350»m.

La poudre congelée ainsi obtenue est laissée à température ambiante jusqu'à ce qu'elle atteigne 4°C. Puis on la presse dans la presse à vis. On obtient alors environ 160kg de suspension.

Ces 160kg de suspension sont osmosés à -2°C jusqu'à obtenir 5kg de rétentat. Le rétentat est ensuite réparti en couches de 2cm d'épaisseur dans des plateaux et placé au congélateur à -25°C.

La plante a été cependant remise au congélateur jusqu'à ce qu'elle revienne à la température de -25°C. On la mélange alors à 160kg d'eau purifiée préalablement refroidie à 4°C et on attend à température ambiante que la plante atteigne à nouveau 4°C. On réalise alors le deuxième pressage. On obtient 180kg de suspension que l'on osmose jusqu'à obtenir 5kg de rétentat placés au congélateur dans les mêmes conditions que précédemment.

On réalise ensuite la même opération encore deux fois. On obtient successivement 140kg et 130kg de suspension et en finale 20kg de rétentat qui sont ensuite filtrés sur filtre (grille) de porosité 100»m, et lyophilisés. Le rendement final en matière sèche lyophilisée est de 12kg.

Etant donné que la plante de départ contenait 84% d'eau le rendement par rapport à la matière sèche de départ est de 37,5%.

La teneur en protoplaste peut être estimée comme étant la fraction de la suspension qui est retenue sur des filtres de porosité 0,45»m et qui passe sur des filtres de porosité 100»m; dans ce cas on peut estimer la teneur en protoplastes à 12% de la matière sèche totale le reste de la matière sèche est représentée par des substances en solution et des substances issues de la destruction mécanique des cellules.

### - Exemple 2

200kg de somités fleuries fraîches de Spirea ulmaria sont récoltés et placés en couches de 10cm environ dans une chambre froide à -25°C. Après 16 heures, la drogue est broyée dans un broyeur à couteaux pour donner des morceaux de 1 à quelques cm.

Puis la drogue coupée est admise dans le cryobroyeur et amenée à une température de -80°C. La température du broyeur est régulée à -100°C et le tamis possède une porosité de 450»m.

La poudre congelée ainsi obtenue est laissée à température ambiante jusqu'à ce qu'elle atteigne 4°C. Puis on la presse dans la presse à vis. On obtient alors environ 105kg de suspension.

Ces 105kg de suspension sont osmosés à -1°C jusqu'à obtenir 5kg de rétentat. Le rétentat est ensuite réparti en couches de 2cm d'épaisseur dans des plateaux et placé au congélateur à -25°C.

La plante a été cependant remise au congélateur jusqu'à ce qu'elle revienne à la température de -25°C. On la mélange alors à 105kg d'eau purifiée préalablement refroidie à 4°C et on attend à température ambiante que la plante atteigne à nouveau 4°C. On réalise alors le deuxième pressage. On obtient 155kg de suspension que l'on osmose jusqu'à obtenir 5kg de rétentat placés au congélateur dans les mêmes conditions que précédemment.

On réalise ensuite la même opération encore deux fois. On obtient successivement 120kg et 150kg de suspension et en finale 20kg de rétentat qui sont ensuite filtrés sur filtre (grille) de porosité 100»m, et lyophilisés. Le rendement final en matière sèche lyophilisée est de 9kg.

Etant donné que la plante de départ contenait 78% d'eau le rendement par rapport à la matière sèche de départ est de 20,45%.

La teneur en protoplaste peut être estimée comme étant la fraction de la suspension qui est retenue sur des filtres de porosité 0,45»m et qui passe sur des filtres de porosité 100»m. Dans ce cas on peut estimer la teneur en protoplastes à 17% de la matière sèche totale le reste de la matière sèche est représentée par des substances en solution et des substances issue de la destruction mécanique des cellules.

### - Exemple 3

200kg de parties aériennes de Fumaria officinalis fraîches sont récoltés et placés en couches de 5cm environ dans une chambre froide à -25°C. Après 22 heures, la drogue est broyée dans un broyeur à couteaux pour donner des morceaux de 1 à quelques cm.

Puis la drogue coupée est admise dans le cryobroyeur et amenée à une température de -70°C. La température du broyeur est régulée à -90°C et le tamis possède une porosité de 500»m.

La poudre congelée ainsi obtenue est laissée à température ambiante jusqu'à ce qu'elle atteigne 4°C. Puis on la presse dans la presse à vis. On obtient alors environ 155kg de suspension.

Ces 155kg de suspension sont osmosés à 2°C jusqu'à obtenir 5kg de rétentat. Le rétentat est ensuite réparti en couches de 2cm d'épaisseur dans des plateaux et placés au congélateur à -25°C.

La plante a été cependant remise au congélateur jusqu'à ce qu'elle revienne à la température de -25°C. On la mélange alors à 205kg d'eau purifiée préalablement refroidie à 4°C et on attend à température ambiante que la plante atteigne à nouveau 4°C. On réalise alors le deuxième pressage. On obtient 125kg de suspension que l'on osmose jusqu'à obtenir 5kg de rétentat placés au congélateur dans les mêmes conditions que précédemment.

On réalise ensuite la même opération encore deux fois. On obtient successivement 100kg et 135kg de suspension et en finale 20kg de rétentat qui sont ensuite filtrés sur filtre (grille) de porosité 100»m, et qui sont ensuite lyophilisée. Le rendement final en matière sèche lyophilisée est de 10kg.

Etant donné que la plante de départ contenait 82% d'eau le rendement par rapport à la matière sèche de départ est de 21,77%.

La teneur en protoplaste peut être estimée comme étant la fraction de la suspension qui est retenue sur des filtres de porosité 0,45»m et qui passe sur des filtres de porosité 100»m. Dans ce cas on peut estimer la teneur en protoplastes à 11% de la matière sèche totale le reste de la matière sèche est représentée par des substances en solution et des substances issues de la destruction mécanique des cellules.

## Revendications

1. Composition végétale sèche issue d'une plante, cette composition étant exempte de cellulose et formée de protoplastes en mélange avec des constituants hydrosolubles appartenant au suc de cette plante, pour reconstituer l'essentiel du suc du végétal dont cette composition est issue lorsqu'elle est mise en suspension dans de l'eau

2. Composition selon la revendication 1, caractérisée en ce qu'elle se présente sous la forme d'un lyophilisat.

3. Composition végétale selon la revendication 1 ou la revendication 2, caractérisée en ce qu'elle comprend des protoplastes, d'un diamètre compris entre 0,45»m et 100»m, insolubles dans l'eau.

4. Composition selon l'une des revendications 1 à 3, caractérisée en ce que la teneur en protoplastes est d'environ 10 à 20% en poids sec.

5. Procédé d'obtention d'une composition selon l'une des revendications 1 à 4, caractérisé en ce qu'il comprend:
- un premier broyage de la plante entière, ou de parties de cette plante, à une température comprise entre -10°C et -40°C, de préférence à environ -25°C,
- un second broyage des fractions de plantes obtenues lors de l'étape de broyage précédente, à une température comprise entre -40°C et -100°C, de préférence à environ -70°C,
- le tamisage des fractions obtenues lors de l'étape de broyage précédente, maintenues à une température comprise entre -40°C et -100°C, à l'aide de tamis de granulométrie variant d'environ 100»m à environ 500»m, suivi de la récupération des fractions passées à travers ce tamis,
- le pressage des fractions récupérées à l'étape précédente, et ramenées à une température de 0°C ± 5°C; les fractions pressées sont ensuite soumises au cycle d'opérations suivantes:
. congélation à une température comprise entre -10°C et -40°C, de préférence à environ -25°C,
. mise en suspension de ces fractions congelées dans une quantité d'eau approximativement égale à la quantité de liquide obtenu lors de l'étape de pressage précédente,
. pressage des fractions en suspension dans l'eau et ramenées à une température de 0°C ± 5°C, ce cycle étant répété entre 2 et 6 fois, de préférence 4 fois,
- la filtration des quantités de liquides obtenus lors de chacune des étapes de pressage précédentes, ces quantités étant, le cas échéant, réunies en une solution unique, sur un filtre ayant une porosité de 100»m, et récupération des filtrats (ou du filtrat)
- la concentration des filtrats (ou du filtrat) par une méthode d'élimination de l'eau à froid, notamment par osmose inverse, à la température de 0°C ± 5°C,
- avantageusement, la congélation des solutions (ou de la solution) concentrées obtenues à l'étape précédente, à une température de -10°C à -40°C, notamment de l'ordre de -25°C, suivie d'une étape de lyophilisation ce qui conduit à l'obtention d'une composition selon l'une des revendications 1 à 4 sous forme de poudre sèche.

6. Composition pharmaceutique caractérisée en ce qu'elle comprend une composition selon l'une des revendications 1 à 4 en association avec un véhicule physiologiquement acceptable.

7. Composition cosmétique caractérisée en ce qu'elle comprend une composition selon l'une des revendications 1 à 4 en association avec un véhicule physiologiquement acceptable.

8. Composition selon la revendication 6 ou la revendication 7, caractérisée en ce qu'elle se présente sous forme de gélules, comprimés, gel, émulsion ou pommade.

9. Composition alimentaire ou diététique, caractérisée en ce qu'elle comprend une composition selon l'une des revendications 1 à 4.

## Patentansprüche

1. Trockene, pflanzliche Zusammensetzung pflanzlichen Ursprungs, die frei von Cellulose ist und von Protoplasten in Mischung mit wasserlöslichen, zu dem Saft der Pflanze gehörenden Bestandteilen gebildet ist, zur Rekonstitution des Wesentlichen des dieser Zusammensetzung zugrundeliegenden Pflanzensaftes bei Suspension derselben in Wasser.

2. Zusammensetzung nach Anspruch 1, dadurch gekennzeichnet, daß die in Form eines Lyophilisats vorliegt.

3. Pflanzliche Zusammensetzung nach Anspruch 1 oder 2, dadurch gekennzeichnet, daß sie in Wasser unlösliche Protoplasten mit einem Durchmesser zwischen 0,45 »m und 100»m enthält.

4. Zusammensetzung nach einem der Ansprüche 1 bis 3, dadurch gekennzeichnet, daß der Gehalt an Protoplasten etwa 10 bis 20 %, bezogen auf Trockengewicht, beträgt.

5. Verfahren zur Herstellung einer Zusammensetzung nach einem der Ansprüche 1 bis 4, gekennzeichnet durch die Schritte
- erstes Zerkleinern der gesamten Pflanze oder von Teilen dieser Pflanze bei einer Temperatur zwischen -10°C und -40°C, vorzugsweise bei etwa -25°C,
- zweites Zerkleinern der erhaltenen Pflanzenfraktionen im Anschluß an die vorherige Zerkleinerungsstufe bei einer Temperatur zwischen -40°C und -100°C, vorzugsweise bei etwa -70°C,
- Sieben der erhaltenen Fraktionen nach der vorhergehenden Zerkleinerungsstufe, wobei diese bei einer Temperatur zwischen -40°C und -100°C gehalten werden, mit Hilfe von verschiedenen Sieben für die Granulometrie von etwa 100»m bis etwa 500»m, gefolgt von der Sammlung von durch das Sieb gehenden Fraktionen,
- Pressen der in der vorherigen Stufe gewonnenen und auf eine Temperatur von 0°C ± 5°C gebrachten Fraktionen;
wobei die geßrepten Fraktionen anschließend einen Zyklus der folgenden Maßnahmen unterworfen werden:
. Einfrieren auf eine Temperatur zwischen -10°C und -40°C, vorzugsweise von etwa -25°C,
. Suspendierung dieser eingefrorenen Fraktionen in einer Wassermenge, die etwa gleich der in der vorherigen Stufe des Pressens erhaltenen Flüssigkeitsmenge ist,
. Pressen der in Wasser suspendierten und auf eine Temperatur von 0°C ± 5°C gebrachten Fraktionen, wobei dieser Zyklus zwischen 2 und 6, vorzugsweise 4 mal wiederholt wird,
- Filtration der nach einer jeden der vorherigen Stufen erhaltenen Flüssigkeitsmengen, wobei diese Mengen gegebenenfalls zu einer einzigen Lösung vereint werden, durch einen Filter mit einer Porosität von 100»m, und Sammlung des Filtrats bzw. der Filtrate,
- Konzentration des Filtrats bzw. der Filtrate mittels eines Verfahrens zum Ausschluß von Wasser in der Kälte, insbesondere der Umkehrosmose, bei einer Temperatur von 0°C bis ± 5°C,
- vorzugsweise Einfrieren der in der vorherigen Stufe erhaltenen, konzentrierten Lösung(en) bei einer Temperatur von -10°C bis -40°C, insbesondere in der Größenordnung von -25°C, gefolgt von einer Stufe der Lyophilisation, wodurch eine Zusammensetzung gemäß einem der Ansprüche 1 bis 4 in Form eines trockenen Pulvers erhalten wird.

6. Pharmazeutische Zusammensetzung, dadurch gekennzeichnet daß sie eine Zusammensetzung gemäß einem der Ansprüche 1 bis 4 zusammen mit einem physiologisch verträglichen Träger enthält.

7. Kosmetische Zusammensetzung, dadurch gekennzeichnet, daß sie eine Zusammensetzung gemäß einem der Ansprüche 1 bis 4 zusammen mit einem physiologisch verträglichen Träger enthält.

8. Zusammensetzung nach Anspruch 6 oder 7, dadurch gekennzeichnet, daß sie in Form von Kapseln, Tabletten, Gelen, Emulsionen oder Salben vorliegt.

9. Ernährungszusammensetzung oder diätetische Zusammensetzung, dadurch gekennzeichnet, daß sie eine Zusammensetzung gemäß einem der Ansprüche 1 bis 4 enthält.

## Claims

1. Dried plant composition derived from a plant, this composition being free of cellulose and made up of protoplasts in the form of a mixture with water-soluble constituents belonging to the juice of this plant, for reconstituting the bulk of the plant juice from which this composition is derived when the said composition is suspended in water.

2. Composition according to Claim 1, characterized in that it is provided in the form of a freeze-dried product.

3. Plant composition according to Claim 1 or Claim 2, characterized in that it comprises protoplasts, with a diameter of between 0.45 »m and 100 »m, which are insoluble in water.

4. Composition according to one of Claims 1 to 3, characterized in that the protoplast content is about 10 to 20% on dry weight basis.

5. Process for producing a composition according to one of Claims 1 to 4, characterized in that it comprises:
- a first grinding of the whole plant, or of parts of this plant, at a temperature of between -10°C and -40°C, preferably at about -25°C,
- a second grinding of the plant fractions obtained during the preceding grinding step, at a temperature of between -40°C and -100°C, preferably at about -70°C,
- sieving the fractions obtained during the preceding grinding step, maintained at a temperature of between -40°C and -100°C, by means of a sieve offering a particle size ranging from about 100 »m to about 500 »m, followed by the recovery of the fractions which passed through this sieve,
- pressing the fractions recovered in the preceding step and adjusted to a temperature of 0°C ± 5°C; the pressed fractions are then subjected to the following cycle of operations:
. freezing at a temperature of between -10°C and -40°C, preferably at about -25°C,
. suspending these frozen fractions in a quantity of water approximately equal to the quantity of liquid obtained during the preceding pressing step,
. pressing the fractions suspended in water and adjusted to a temperature of 0°C ± 5°C, this cycle being repeated between 2 and 6 times, preferably 4 times,
- filtering the quantities of liquid obtained during each of the preceding pressing steps, these quantities being, where appropriate, pooled into a single solution, on a filter having a porosity of 100 »m, and recovering the filtrates (or the filtrate)
- concentrating the filtrates (or the filtrate) by a method for removing water at cold temperature, especially by reverse osmosis, at the temperature of 0°C ± 5°C,
- advantageously, freezing the concentrated solutions (or solution) obtained in the preceding step, at a temperature of -10°C to -40°C, especially of the order of -25°C, followed by a freeze-drying step, thereby resulting in the production of a composition according to one of Claims 1 to 4 in the form of a dry powder.

6. Pharmaceutical composition characterized in that it comprises a composition according to one of Claims 1 to 4, combined with a physiologically acceptable vehicle.

7. Cosmetic composition characterized in that it comprises a composition according to one of Claims 1 to 4, combined with a physiologically acceptable vehicle.

8. Composition according to Claim 6 or Claim 7, characterized in that it is provided in the form of hard gelatin capsules, tablets, gel, emulsion or ointment.

9. Food or dietary composition, characterized in that it comprises a composition according to one of Claims 1 to 4.
